# EUROPEAN PATENT APPLICATION

(11) **EP 1 604 675 A1**
(43) Date of publication of application: **14.12.2005**
(21) Application number: 04255524.3
(22) Date of filing: 13.09.2004
(51) Int. Cl.: A61K 35/78, A23L 1/30, A61P 29/00

(54) **Morinda citrifolia-based formulation as lipoxygenase inhibitor**

(30) Priority: 07.06.2004 US 862828
(71) Applicant: Morinda, Inc., Provo, UT 84604 (US)
(72) Inventor: Palu, Afa Kehaati, Orem, UT 84058 (US); Zhou, Bing-Nan, Pleasant Grove, UT 84062 (US); Su, Chen, West Jordan, UT 84084 (US); Jensen, Claude Jarakae, Cedar Hills, UT 84062 (US); Story, Stephen, Alpine, UT 84004 (US)
(74) Representative: Gemmell, Peter Alan

(57) **Abstract**

The present invention is directed to methods and formulations for inhibiting Lipoxygenase enzymes that function to biosynthesize or metabolize arachidonic acid into its intermediate Leukotriene constituents, as well as a method and formulation for treating and preventing diseases, including inflammatory diseases, and the symptoms associated with such diseases. The present invention methods and formulations effectively function as such through the introduction into the body (e.g. ingesting) a safe, pre-determined dosage of a naturaceutical composition formulated with or comprising one or more processed *Morinda citrifolia* products for a safe, pre-determined duration, wherein the processed *Morinda citrifolia* product may comprise one or more isolated active ingredients.

## Description

### BACKGROUND OF THE INVENTION

### 1. Field of the Invention

The present invention relates to composition formulated as an inhibitor of 5-Lipoxygenase (5-LOX) and 15-Lipoxygenase (15-LOX), wherein the composition is formulated with one or more processed or unprocessed *Morinda citrifolia* ingredients or products as derived from the Indian Mulberry plant.

### 2. Background and Related Art

Eicosanoids are continuously synthesized in membranes from 20-carbon fatty acid chains that contain at least three double bonds. There are four major classes of eicosanoids - prostaglandins, prostacyclins, thromboxanes, and leukotrienes, and they are all made mainly from arachidonic acid. The synthesis of all but the leukotrienes involves the enzyme cyclooxygenase (COX); the synthesis of leukotrienes involves the enzyme lipoxygenase (LOX). These synthetic pathways are targets for a large number of therapeutic drugs, since eicosanoids play an important part in pain, fever, and inflammation. Corticosteroid hormones such as cortisone, for example, which inhibit the activity of the phospholipase in the first step of the eicosanoid synthesis pathway shown, are widely used clinically to treat noninfectious inflammatory diseases, such as some forms of arthritis. Nonsteroid anti-inflammatory drugs such as aspirin and ibuprofen, by contrast, block the first oxidation step, which is catalyzed by cyclooxygenase. Certain prostaglandins that are produced in large amounts in the uterus at the time of childbirth to stimulate the contraction of the uterine smooth muscle cells are widely used as pharmacological agents to induce abortion.

The enzymes of the 5-LOX and 15-LOX pathway produce active metabolites from arachidonic acid that cause inflammation. This has been shown both by the identification of higher levels of leukotrienes in both acute and chronic inflammatory lesions coupled with the evidence of primary signs of inflammation when leukotrienes are added to tissue cultures. Leukotrienes are a family of lipid mediators involved in acute and chronic inflammation and allergic response diseases. They are the biologically active metabolites of arachidonic acid and have been implicated in the pathological manifestations of inflammatory diseases, including asthma, arthritis, psoriasis, and inflammatory bowel disease. The biosynthesis of leukotrienes (LT or LT's) begins with the oxygenation of arachidonic acid into an unstable epoxide known as LTA₄ (an intermediate central to the formation of leukotrienes) by the enzyme 5-lipoxygenase (5-LOX). LTA₄ can further be converted into the potent chemo attractant LTB₄ by the enzyme LTA₄ hydrolase or conjugated with glutathione (GSH) to produce LTC₄ by a specific microsomal GSH S-transferase (MGST) known as LTC₄ synthetase (LTC₄S). LTC₄ is the parent compound of the cysteinyl-leukotrienes (cys-LTs) that include LTC₄, LTD₄, and LTE₄. These three cysteinyl-leukotrienes are potent smooth muscle constricting agents, particularly in the respiratory and circulatory systems. These are mediated via at least two cell receptors, CysLT1 and CysLT2. The CysLT1 receptor is a G-protein-coupled receptor with seven transmembrane regions. There has been numerous amounts of data that has been collected, which clearly demonstrates that the CysLT's play a pivotal role in inflammatory and allergic response diseases, particularly asthma.

It has also been established that these lipid mediators have profound hemodynamic effects, constricting coronary blood vessels, resulting in a reduction of cardiac output efficiency. Moreover, CysLT's have been shown to induce the secretion of *von Willebrand* factor and surface expression of P-selectin in cultured HUVEC. *Von Willebrand* is a genetic disorder. The most common types, and those most familiar to people, are the hemophiliac diseases. These enzymes of the 5-LOX pathway produce active metabolites from arachidonic acid that cause inflammation. This has been shown both by the identification of higher levels of leukotrienes in both acute and chronic inflammatory lesions coupled with the evidence of primary signs of inflammation when leukotrienes are added to tissue cultures.

In addition, the cysteinyl LT's are predominantly secreted by eosinophils, mast cells, and macrophages, which cause vasodilatation, increase postcapillary venule permeability, and stimulate bronchoconstriction and mucous secretion. Furthermore, it has been observed that elevated leukotriene LTC₄ synthase activity was observed in peripheral blood granulocyte suspensions from patients with chronic myeloid leukemia (CML), and human bone marrow-derived myeloid progenitor cells. In asthma, the cysteinyl leukotrienes are present in alveolar lavage fluid of patients. Therefore, the presence of 5-LOX and leukotriene synthase are clinically important in the diagnosis of patients with bronchial asthma.

### SUMMARY OF THE INVENTION

The present invention is directed to methods of and formulations to inhibit the oxygenation and metabolizing of arachidonic acid into its leukotriene synthesized intermediates by inhibiting 5-Lipoxygenase (5-LOX), 15-Lipoxygenase (15-LOX) and the lipid mediators known as leukotrienes that contribute to the pathological manifestations of inflammatory diseases, namely, asthma, arthritis, psoriasis, and inflammatory bowel disease, as well as the treatment and prevention of these diseases through the introduction into the body a safe, pre-determined dosage of a composition formulated with or comprising one or more processed or unprocessed *Morinda citrifolia* ingredients or products for a safe, pre-determined duration.

The present invention naturaceutical composition comprises at least one processed *Morinda citrifolia* product in one of its several forms (preferably the fruit juice), formulated with or without other ingredients, either natural or artificial, as needed. In a currently preferred embodiment, a quantity of a processed *Morinda citrifolia* product is obtained in the form of fruit juice, puree juice or juice puree, pulp, seed oil, and/or dietary fiber, using the process(es) as described below. Subsequently, an amount of any one of or a combination of these is formulated with other ingredients to create a naturaceutical composition formulated to provide significant health advantages and to assist in the treatment of and provide preventative effects for inflammatory diseases through the inhibition of 5-LOX and 15-LOX. In another preferred embodiment the naturaceutical composition is a liquid that may be administered orally or through intravenous injection, wherein the active ingredients, namely *Morinda citrifolia,* are allowed to be absorbed into the tissues to inhibit Lipoxygenase and reduce/regulate leukotriene production.

The present invention further features a method of inhibiting 5-LOX and 15-LOX and treating, inhibiting, preventing, and reversing inflammatory diseases through the prophylactic administration of a naturaceutical composition comprising at least one processed *Morinda citrifolia* product as an active ingredient.

These and other features and advantages of the present invention will be set forth or will become more fully apparent in the description that follows and in the appended claims. The features and advantages may be realized and obtained by means of the instruments and combinations particularly pointed out in the appended claims. Furthermore, the features and advantages of the invention may be learned by the practice of the invention or will be obvious from the description, as set forth hereinafter.

### DETAILED DESCRIPTION OF THE INVENTION

It will be readily understood that the components of the present invention, as generally described herein, could be arranged and designed in a wide variety of different configurations. Thus, the following more detailed description of the embodiments of the system and method of the present invention is not intended to limit the scope of the invention, as claimed, but is merely representative of the presently preferred embodiments of the invention. The scope of the invention is, therefore, indicated by the appended claims rather than by the foregoing description. All changes that come within the meaning and range of equivalency of the claims are to be embraced within their scope.

It will be appreciated by those of ordinary skill in the art that the objects of this invention can be achieved without the expense of undue experimentation using well known variants, modifications, or equivalents of the methods and techniques described herein. The skilled artisan will also appreciate that alternative means, other than those specifically described, are available in the art to achieve the functional features of the molecules described herein and how to employ those alternatives to achieve functional equivalents of the molecules of the present invention. It is intended that the present invention include those variants, modifications, alternatives, and equivalents which are appreciated by the skilled artisan and encompassed by the spirit and scope of the present disclosure.

The present invention describes and features a method and formulation for inhibiting 5-lipoxygenase (5-LOX), 15-lipoxygenase (15-LOX) and for treating and preventing mammalian inflammatory diseases through the administration of a naturaceutical formulation comprising at least one *Morinda citrifolia* product in processed form.

The presently preferred embodiments of the invention will be best understood, and its benefits and advantages more clearly pointed out, by separating the following more detailed description into sections. The first section of the detailed description is a general discussion regarding *Morinda citrifolia,* including its origins, processing techniques, and health benefits. The second section of the detailed description discusses some of the methods employed to produce and manufacture the processed *Morinda citrifolia* products. The third section of the detailed description contains a discussion regarding naturaceutical formulations and compositions comprising *Morinda citrifolia* product used to inhibit 5-LOX, 15-LOX and to treat and prevent diseases, including inflammatory diseases, as well as a description of methods for administering said *Morinda citrifolia* product. Finally, the fourth section discusses preventative and treatment effects of the processed *Morinda citrifolia* products on 5-LOX, 15-LOX, as well as the preventative and treatment effects of *Morinda citrifolia* against disease, including inflammatory diseases. Examples of experimental studies and the results obtained are also provided.

### GENERAL DESCRIPTION OF MORINDA CITRIFOLIA

Embodiments of the present invention include a formulation comprising one or more forms of processed *Morinda citrifolia* for inhibiting 5-lipoxygenase (5-LOX), 15-lipoxygenase (15-LOX) and for treating and preventing mammalian inflammatory diseases through the administration of a naturaceutical formulation comprising at least one *Morinda citrifolia* product in processed fonn. Accordingly, the following is a general description of *Morinda citrifolia,* including its origins, processing techniques, and health benefits. A more detailed description of the *Morinda citrifolia-based* formulations and compositions used to treat mammalian inflammatory diseases and the methods used for administering these to a subject, including examples of experimental studies and the results attained, is provided below.

The Indian Mulberry or Morinda citrifolia plant, known scientifically as *Morinda Citrifolia L.* ("*Morinda citrifolia*"), is a shrub or small tree up to 10 m in height. The leaves are oppositely arranged with an elliptic to ovate form. The small white flowers are contained in a fleshy, globose, head-like cluster. The fruits are large, fleshy, and ovoid. At maturity, they are creamy-white and edible, but have an unpleasant taste and odor. The plant is native to Southeast Asia and has spread in early times to a vast area from India to eastern Polynesia. It grows randomly in the wild, and it has been cultivated in plantations and small individual growing plots. The *Morinda citrifolia* flowers are small, white, three to five lobed, tubular, fragrant, and about 1.25 cm long. The flowers develop into compound fruits composed of many small drupes fused into an ovoid, ellipsoid or roundish, lumpy body, with waxy, white, or greenish-white or yellowish, semi-translucent skin. The fruit contains "eyes" on its surface, similar to a potato. The fruit is juicy, bitter, dull-yellow or yellowish-white, and contains numerous red-brown, hard, oblong-triangular, winged 2-celled stones, each containing four seeds.

When fully ripe, the fruit has a pronounced odor like rancid cheese. Although the fruit has been eaten by several nationalities as food, the most common use of the *Morinda citrifolia* plant was as a red and yellow dye source. Recently, there has been an interest in the nutritional and health benefits of the *Morinda citrifolia* plant, further discussed below.

Because the *Morinda citrifolia* fruit is for all practical purposes inedible, the fruit must be processed in order to make it palatable for human consumption and included in the naturaceutical used to inhibit 5-LOX and treat various inflammatory diseases, such as arthritis, asthma, etc.. Processed *Morinda citrifolia* fruit juice can be prepared by separating seeds and peels from the juice and pulp of a ripened *Morinda citrifolia* fruit; filtering the pulp from the juice; and packaging the juice. Alternatively, rather than packaging the juice, the juice can be immediately included as an ingredient in another food product, frozen or pasteurized. In some embodiments, the juice and pulp can be pureed into a homogenous blend to be mixed with other ingredients. Other process include freeze drying the fruit and juice. The fruit and juice can be reconstituted during production of the final juice product. Still other processes include air drying the fruit and juices, prior to being masticated.

The present invention utilizes the fruit juice and the oil extracted from the *Morinda Citrifolia* plant. In a currently preferred process of producing *Morinda citrifolia* fruit juice, the fruit is either hand picked or picked by mechanical equipment. The fruit can be harvested when it is at least one inch (2-3 cm) and up to 12 inches (24-36 cm) in diameter. The fruit preferably has a color ranging from a dark green through a yellow-green up to a white color, and gradations of color in between. The fruit is thoroughly cleaned after harvesting and before any processing occurs.

The fruit is allowed to ripen or age from 0 to 14 days, with most fruit being held from 2 to 3 days. The fruit is ripened or aged by being placed on equipment so it does not contact the ground. It is preferably covered with a cloth or netting material during aging, but can be aged without being covered. When ready for further processing the fruit is light in color, from a light green, light yellow, white or translucent color. The fruit is inspected for spoilage or for excessively green color and hard firmness. Spoiled and hard green fruit is separated from the acceptable fruit.

The ripened and aged fruit is preferably placed in plastic lined containers for further processing and transport. The containers of aged fruit can be held from 0 to 30 days. Most fruit containers are held for 7 to 14 days before processing. The containers can optionally be stored under refrigerated conditions prior to further processing. The fruit is unpacked from the storage containers and is processed through a manual or mechanical separator. The seeds and peel are separated from the juice and pulp.

The juice and pulp can be packaged into containers for storage and transport. Alternatively, the juice and pulp can be immediately processed into finished juice product. The containers can be stored in refrigerated, frozen, or room temperature conditions. The *Morinda citrifolia* juice and pulp are preferably blended in a homogenous blend, after which they may be mixed with other ingredients, such as flavorings, sweeteners, nutritional ingredients, botanicals, and colorings. The finished juice product is preferably heated and pasteurized at a minimum temperature of 181° F (83° C) or higher up to 212° F (100° C). Another product manufactured is *Morinda citrifolia* puree and puree juice, in either concentrate or diluted form. Puree is essentially the pulp a separated from the seeds and is different than the fruit juice product described herein.

The product is filled and sealed into a final container of plastic, glass, or another suitable material that can withstand the processing temperatures. The containers are maintained at the filling temperature or may be cooled rapidly and then placed in a shipping container. The shipping containers are preferably wrapped with a material and in a manner to maintain or control the temperature of the product in the final containers.

The juice and pulp may be further processed by separating the pulp from the juice through filtering equipment. The filtering equipment preferably consists of, but is not limited to, a centrifuge decanter, a screen filter with a size from 1 micron up to 2000 microns, more preferably less than 500 microns, a filter press, reverse osmosis filtration, and any other standard commercial filtration devices. The operating filter pressure preferably ranges from 0.1 psig up to about 1000 psig. The flow rate preferably ranges from 0.1 g.p.m. up to 1000 g.p.m., and more preferably between 5 and 50 g.p.m. The wet pulp is washed and filtered at least once and up to 10 times to remove any juice from the pulp. The wet pulp typically has a fiber content of 10 to 40 percent by weight. The wet pulp is preferably pasteurized at a temperature of 181° F (83° C) minimum and then packed in drums for further processing or made into a high fiber product.

The method for extracting and processing the oil is described in co-pending Application Serial No. 09/384,785, filed on August 27, 1999, which is incorporated by reference herein. The *Morinda citrifolia* oil typically includes a mixture of several different fatty acids as triglycerides, such as palmitic, stearic, oleic, and linoleic fatty acids, and other fatty acids present in lesser quantities. In addition, the oil preferably includes an antioxidant to inhibit spoilage of the oil. Conventional food grade antioxidants are preferably used.

The *Morinda citrifolia* plant is rich in natural ingredients. Those ingredients that have been discovered include: from the leaves: alanine, anthraquinones, arginine, ascorbic acid, aspartic acid, calcium, beta-carotene, cysteine, cystine, glycine, glutamic acid, glycosides, histidine, iron, leucine, isoleucine, methionine, niacin, phenylalanine, phosphorus, proline, resins, riboflavin, serine, beta-sitosterol, thiamine, threonine, tryptophan, tyrosine, ursolic acid, and valine; from the flowers: acacetin-7-o-beta-d (+)-glucopyranoside, 5,7-dimethyl-apigenin-4'-o-beta-d(+)-galactopyranoside, and 6,8-dimethoxy-3-methylanthraquinone-1-o-beta-rhamnosyl-glucopyranoside; from the fruit: acetic acid, asperuloside, butanoic acid, benzoic acid, benzyl alcohol, 1-butanol, caprylic acid, decanoic acid, (E)-6-dodeceno-gamma-lactone, (Z,Z,Z)-8,11,14-eicosatrienoic acid, elaidic acid, ethyl decanoate, ethyl hexanoate, ethyl octanoate, ethyl palmitate, (Z)-6-(ethylthiomethyl) benzene, eugenol, glucose, heptanoic acid, 2-heptanone, hexanal, hexanamide, hexanedioic acid, hexanoic acid (hexoic acid), 1-hexanol, 3-hydroxy-2-butanone, lauric acid, limonene, linoleic acid, 2-methylbutanoic acid, 3-methyl-2-buten-1-ol, 3-methyl-3-buten-1-ol, methyl decanoate, methyl elaidate, methyl hexanoate, methyl 3-methylthio-propanoate, methyl octanoate, methyl oleate, methyl palmitate, 2-methylpropanoic acid, 3-methylthiopropanoic acid, myristic acid, nonanoic acid, octanoic acid (octoic acid), oleic acid, palmitic acid, potassium, scopoletin, undecanoic acid, (Z,Z)-2,5-undecadien-1-ol, and vomifol; from the roots: anthraquinones, asperuloside (rubichloric acid), damnacanthal, glycosides, morindadiol, morindine, morindone, mucilaginous matter, nor-damnacanthal, rubiadin, rubiadin monomethyl ether, resins, soranjidiol, sterols, and trihydroxymethyl anthraquinone-monomethyl ether; from the root bark: alizarin, chlororubin, glycosides (pentose, hexose), morindadiol, morindanigrine, morindine, morindone, resinous matter, rubiadin monomethyl ether, and soranjidiol; from the wood: anthragallol-2,3-dimethylether; from the tissue culture: damnacanthal, lucidin, lucidin-3-primeveroside, and morindone-6beta-primeveroside; from the plant: alizarin, alizarin-alpha-methyl ether, anthraquinones, asperuloside, hexanoic acid, morindadiol, morindone, morindogenin, octanoic acid, and ursolic acid.

Recently, many health benefits have been discovered stemming from the use of products containing *Morinda citrifolia.* One benefit of *Morinda citrifolia* is found in its ability to isolate and produce Xeronine, which is a relatively small alkaloid physiologically active within the body. Xeronine occurs in practically all healthy cells of plants, animals and microorganisms. Even though *Morinda citrifolia* has a negligible amount of free Xeronine, it contains appreciable amounts of the precursor of Xeronine, called Proxeronine. Further, *Morinda citrifolia* contains the inactive form of the enzyme Proxeroninase which releases Xeronine from Proxeronine. A paper entitled, "The Pharmacologically Active Ingredient of Morinda citrifolia" by R. M. Heinicke of the University of Hawaii, indicates that *Morinda citrifolia* is "the best raw material to use for the isolation of xeronine," because of the building blocks of Proxeronine and Proxeroninase. These building blocks aid in the isolation and production of Xeronine within the body.

The function of the essential nutrient Xeronine is fourfold. First, Xeronine serves to activate dormant enzymes found in the small intestines. These enzymes are critical to efficient digestion, calm nerves, and overall physical and emotional energy. Second, Xeronine protects and keeps the shape and suppleness of protein molecules so that they may be able to pass through the cell walls and be used to form healthy tissue. Without these nutrients going into the cell, the cell cannot perform its job efficiently. Without Proxeronine to produce Xeronine our cells, and subsequently the body, suffer. Third, Xeronine assists in enlarging the membrane pores of the cells. This enlargement allows for larger chains of peptides (amino acids or proteins) to be admitted into the cell. If these chains are not used they become waste. Fourth, Xeronine, which is made from Proxeronine, assists in enlarging the pores to allow better absorption of nutrients.

Each tissue has cells which contain proteins which have receptor sites for the absorption of Xeronine. Certain of these proteins are the inert forms of enzymes which require absorbed Xeronine to become active. Thus Xeronine, by converting the body's procollagenase system into a specific protease, quickly and safely removes the dead tissue from skin. Other proteins become potential receptor sites for hormones after they react with Xeronine. Thus the action of *Morinda citrifolia* in making a person feel well is probably caused by Xeronine converting certain brain receptor proteins into active sites for the absorption of the endorphin, the well being hormones. Other proteins form pores through membranes in the intestines, the blood vessels and other body organs. Absorbing Xeronine on these proteins changes the shape of the pores and thus affects the passage of molecules through the membranes.

Because of its many benefits, *Morinda citrifolia* has been known to provide a number of anecdotal effects in individuals having cancer, arthritis, headaches, indigestion, malignancies, broken bones, high blood pressure, diabetes, pain, infection, asthma, toothaches, blemishes, immune system failure, and others.

In addition to the numerous health benefits, *Morinda citrifolia* also provides significant benefits to the skin. *Morinda citrifolia* is high in anti-oxidants that help to fight free-radical damage caused by the sun and other changing environmental conditions and elements. To stay healthy, the skin must cope with these elements and conditions and repair the damage caused at the same time. The skin is in a constant state of repair as it sheds the dead cells on the surface and replenishes the lower layers. *Morinda citrifolia* is also especially rich in linoleic acid, which is an essential fatty acid having the specific ability to nourish the health of the skin.

### METHODS USED TO PRODUCE PROCESSED MORINDA CITRIFOLIA PRODUCTS

The methods used to produce processed *Morinda citrifolia* products is described in co-pending Application Serial No. 09/384,785, filed on August 27, 1999, which is incorporated by reference herein. The compositions containing *Morinda citrifolia* may be in a form suitable for oral use, systemic administration, injection, and others. In regards to an oral composition, such a composition may exist, for example, as tablets, or lozenges, aqueous or oily suspensions, dispersible powders or granules, emulsions, syrups or elixirs. Compositions intended for oral use may be prepared according to any method known in the art for the manufacture of *Morinda citrifolia* compositions and such compositions may contain one or more agents selected from the group consisting of sweetening agents, flavoring agents, coloring agents and preserving agents. Tablets contain *Morinda citrifolia* in admixture with non-toxic pharmaceutically acceptable excipients which are suitable for the manufacture of tablets. These excipients may be for example, inert diluents, granulating and disintegrating agents, binding agents, and lubricating agents. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Aqueous suspensions contain the *Morinda citrifolia* in admixture with excipients suitable for the manufacture of aqueous suspensions. Such excipients are suspending agents, for example, sodium carboxymethyl-cellulose, methylcellulose, hydroxy-propylmethycellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethylene-oxycetanol, or condensation products of ethylene oxide with partial esters derived from fatty acids and a hexitol such as polyoxyethylene sorbitor monooleate, or condensation products of ethylene oxide with partial esters derived from fatty acids and hexitol anhydrides, for example polyethylene sorbitan monooleate.

Favorably, this invention provides a method of inhibiting 5-LOX and 15-LOX with a *Morinda citrifolia-*based naturaceutical formulation without any significant tendency to cause gastric or other adverse side effects.

### MORINDA CITRIFOLIA-BASED NATURACEUTICAL FORMULATIONS AND METHODS OF ADMINISTRATION FOR INHIBITING 5-LOX AND 15-LOX

The present invention features methods for introducing an internal composition or formulation to inhibit 5-LOX, 15-LOX and to inhibit the oxygenation of arachidonic acid into its leukotriene intermediate constituents, for the purpose of treating and preventing inflammatory diseases. These methods essentially comprise the introduction of an internal composition into the body of a mammal suffering from one of such diseases. Several embodiments of the internal composition comprising various different ingredients are contemplated for use herein, with each embodiment comprising one or more forms of a processed *Morinda citrifolia* product as taught and explained herein and with optional carrier agent or medium.

The Morinda Citrifolia-based naturaceutical formulations and methods of administration for inhibiting 5-LOX are described in co-pending Application Serial No. 09/384,785, filed on August 27, 1999, which is incorporated by reference herein. The present invention advances treatment of inflammatory diseases by providing a naturaceutical composition formulated with one or more processed *Morinda citrifolia* products derived from the Indian Mulberry plant, which inhibit 5-LOX and 15-LOX metabolism of arachidonic acid. *Morinda citrifolia* is incorporated into various carriers or naturaceutical compositions suitable for *in vivo* treatments. For instance, the inhibitor may be ingested, injected, introduced intravenously, or otherwise internalized as is appropriate and directed.

In one exemplary embodiment, the naturaceutical composition of the present invention comprises one or more of a processed *Morinda citrifolia* product present in an amount by weight between about 0.01 and 100 percent by weight, and preferably between 0.01 and 95 percent by weight. Several embodiments of formulations are included in co-pending Application Serial No. 09/384,785, filed on August 27, 1999, which is incorporated by reference herein. However, these formulations are only intended to be exemplary as one ordinarily skilled in the art will recognize other formulations or compositions comprising the processed *Morinda citrifolia* product.

The processed *Morinda citrifolia* product comprises at least one of the active ingredients in the naturaceutical, or contains one or more active ingredients, such as Quercetin and Rutin, and others, for effectuating the inhibition of 5-LOX, 15-LOX and treating and preventing inflammatory diseases. The Morinda citrifolia product specifically functions to inhibit the oxygenation and metabolizing of arachidonic acid into leukotriene synthesized intermediates or constituents.

Active ingredients within the processed *Morinda citrifolia* product may be extracted using various alcohol or alcohol-based solutions using any known process in the art. Some of the alcohol or alcohol-based solutions include methanol, ethanol, and ethyl acetate. Quercetin and Rutin are active ingredients which may be present within the process or unprocessed *Morinda citrifolia* product.

The processed *Morinda citrifolia* product may be formulated with various other ingredients to produce various compositions, such as a naturaceutical composition, a topical dermal composition, or others. The ingredients to be utilized in a naturaceutical composition are any that are safe for introduction into the body of a mammal, and particularly a human, and may exist in various forms, such as liquids, tablets, lozenges, aqueous or oily solutions, dispersible powders or granules, emulsions, syrups, elixirs, etc. Moreover, since the naturaceutical composition is preferably consumed orally, it may contain one or more agents selected from the group consisting of sweetening agents, flavoring agents, coloring agents, preserving agents, and other medicinal agents as directed.

The ingredients to be utilized in a topical dermal composition are also any that are safe for internalizing into the body of a mammal and may exist in various forms, such as gels, lotions, creams, ointments, etc., each comprising one or more carrier agents. The ingredients for systemically (e.g. intravenously) administered formulations may also comprise any known in the art.

The present invention further features a method of administering a naturaceutical composition to a mammal to inhibit 5-LOX and 15-LOX, thus inhibiting the biosynthesis of leukotrienes, as well as to treat and prevent inflammatory diseases. In one exemplary embodiment, the method comprises the steps of (a) formulating a naturaceutical composition comprising in part a processed *Morinda citrifolia* product, wherein the composition also optionally comprises a carrier, such as water or purified water, and may also comprise other natural or artificial ingredients; (b) administering the naturaceutical composition into the body of a mammal, such that the processed *Morinda citrifolia* product is sufficiently internalized; (c) repeating the above steps as often as necessary to provide an effective amount of the processed *Morinda citrifolia* product to inhibit 5-LOX and 15-LOX therein.

The step of administering the naturaceutical composition into the body preferably comprises ingesting the composition orally through one of several means. Specifically, the naturaceutical composition may be formulated as a liquid, gel, solid, or some other type that would allow the composition to be quickly digested and concentrated within the colon. It is important to note that the step of administering the naturaceutical composition should be carried out in an effective manner so that the greatest concentration of naturaceutical composition is allowed to absorb into the tissues and cells. For the naturaceutical composition to take effect, it must be sufficiently internalized. Once sufficiently internalized, it may then begin to sufficiently inhibit or effectuate the inhibition of 5-LOX and 15-LOX, thus inhibiting the metabolism of arachidonic acid into its leukotriene constituents or active metabolites that adversely function within the inflammatory pathophysiology.

In another embodiment, the step of administering the naturaceutical composition may include injecting the composition into the body using an intravenous pump. This technique is advantageous as it would allow the composition to be localized in the area where it would have the most effect, or the area that would provide for the greatest concentration of the naturaceutical composition.

In one exemplary embodiment, the naturaceutical composition is administered by taking between 1 teaspoon and 2 oz., and preferably 2 oz., of the naturaceutical composition every two hours each day, or at least twice a day on a continued basis. Also, the naturaceutical composition is to be taken on an empty stomach, meaning at a period of time at least two hours prior to consumption of any food or drink. Following this, the naturaceutical composition functions to inhibit 5-LOX and 15-LOX. Of course, one ordinarily skilled in the art will recognize that the amount of composition and frequency of use may vary from individual to individual.

Several embodiments of formulations are included in co-pending Application Serial No. 09/384,785, filed on August 27, 1999, which is incorporated by reference herein. Some of the preferred compositions contemplated by the present invention are comprised of various combinations of: *Morinda citrifolia* puree juice or fruit juice, water, non*-Morinda citrifolia*-based fruit juices, *Morinda citrifolia* dietary fiber, *Morinda citrifolia* oil and oil extract, carrier medium, *Morinda citrifolia* product, *Morinda citrifolia* puree juice concentrate or fruit juice concentrate. As stated, these are only intended as exemplary embodiments and are not to be construed as limiting in any way.

In one exemplary embodiment, the internal composition comprises the ingredients of:
a processed *Morinda citrifolia* product present in an amount by weight between about 10-80 percent; and a carrier medium present in an amount by weight between about 20-90 percent. In this embodiment, the processed *Morinda citrifolia* product may comprise one or more of a processed *Morinda citrifolia* fruit juice, processed *Morinda citrifolia* puree juice, processed *Morinda citrifolia* dietary fiber, and/or processed *Morinda citrifolia* oil extract product.

In another exemplary embodiment, the internal composition comprises the ingredients of: processed *Morinda citrifolia* fruit juice or puree juice present in an amount by weight between about 0.1-80 percent; processed *Morinda citrifolia* oil present in an amount by weight between about 0.1-20 percent; and a carrier medium present in an amount by weight between about 20-90 percent. *Morinda citrifolia* puree juice or fruit juice may also be formulated with a processed *Morinda citrifolia* dietary fiber product present in similar concentrations.

According to the present invention, the particular methods of introducing an internal composition may comprise any method of actually introducing the internal composition into the body of a mammal for the purposes identified herein. Although the particular methods are many, the present invention recognizes that the internal composition may be introduced intravenously, transdermally, orally, or systemically. No matter what method is employed, it is important to thoroughly internalize the composition so that the internal composition, and particularly the *Morinda citrifolia* and other active ingredients, can effectively inhibit 5-LOX, 15-LOX and treat any inflammatory diseases.

The carrier medium identified in the above formulations may comprise any ingredient capable of being introduced into the body of a mammal, and that is also capable of providing the carrying medium to the processed *Morinda citrifolia* product. Specific carrier mediums formulations are well known in the art and not described in detail herein. The purpose of the carrier medium is as stated, to provide a means to embody the processed *Morinda citrifolia* product within the internal composition that is capable of being introduced into the body.

### MORINDA CITRIFOLIA-BASED NATURACEUTICAL ADMINISTRATION PREVENTION AND TREATMENT OF DISEASE

The following examples set forth and present the preventative and treatment effects of the processed *Morinda citrifolia* products on 5-LOX, 15-LOX, as well as the preventative and treatment effects of *Morinda citrifolia* against inflammatory diseases. These examples are not intended to be limiting in any way, but are merely illustrative of the benefits and advantageous, as well as the remedial effects, of the *Morinda citrifolia* products.

There are several main branches of the arachidonic acid cascade that affect inflammation and drug metabolism. Specifically, a first branch of the arachidonic acid cascade results in synthesis by several Cytochrome (CYP) enzymes, namely CYP450, CYP2D6, CYP2C19, CYP3A4, CYP 2C9, and CYP1A2. The second branch is the arachidonic acid cascade into cyclooxygenase-1 and 2 (COX 1 and COX 2). The third branch of the arachidonic acid cascade involves 5-lipoxygenase pathways and its enzymes, such as Leukotriene A₄ (LTA₄) hydrolase and Leukotriene C₄ (LTC₄) synthetase.

Lipoxygenase is required for development of senescence and production of antibacterial compounds in plants. In mammals, lipoxygenase catalyzes the oxidation of arachidonic acid into Eicosanoic acids (5, 12, and 15). Lipoxygenase are enzymes that contain non-heme iron and that use molecular oxygen in the metabolism of arachidonic acid, and also facilitates the biosynthesis of potent mediators, leukotrienes, that have far reaching physiological effects in mammals.

Inflammatory and allergic responses are modulated by arachodonic acid metabolites through a variety of interconnected mechanisms. Important signal molecules (LT's, PG's) in a variety of pathways of inflammation and allergic conditions affect the skin, joints, gastrointestinal, and respiratory systems, in particular asthma. Arachodonic acid is metabolized into various active leukotrienes, namely LTD₄, LTB₄, etc. These are potent inflammatory mediators that contribute to increased mucus production, bronchoconstriction, and eisoniphil infiltration. It has also been shown that products of 5-LOX activities are significant activators of microglia.

The central nervous system comprises neurons and glial cells. Glial cells are supporting cells that provide nutrients and oxygen, insulate one neuron from another, surround and hold neurons in place, and destroy and remove dead neurons. Microglias are the smallest of the glial cells. Microglias become reservoirs for infectious agents, viruses, fungi, etc, which contribute to inflammation. As infected cells in the central nervous system, microglia play a key role in development of various diseases, possibly by the production of toxic factors following infection, or more directly by not providing normal metabolic support for neurons. Moreover, neuroinflammation and oxidative stress are believed to be contributing factors to neurodegeneration in normal aging and in age-related neurological disorders, such as AD, PD, ALS, and Osteoarthritis. Reactive microglias are found in increasing numbers compared to normal tissues. In vitro, stimulated microglia or microglia-like cells secrete neurotoxic materials, and are generators of free radicals through the respiratory burst system. This has led to the hypothesis that activation of these cells could contribute to the death of neuron cells observed in diseased brain tissue. Reactive microglias are round in increasing numbers in the aging brain. They generate large amounts of toxic oxygen free radicals. Any agents that suppress microglia activation are good candidates for protection of neurons.

### Example One: Morinda citrifolia Inhibition of 5-LOX

The present experiment involved the dual inhibition of both cyclooxygenase-2 and 5-lipoxygenase. The COX pathway generates inflammatory PG's, while 5-Lipoxygenase catalyzes the oxidative metabolism of arachidonic acid to 5-hydroxyeicosatetraenoic acid (5-HETE), the initial reaction leading to formation of leukotrienes. Inhibitors of both pathways suppressed neurotoxicity in a dose dependent manner. Both pathway inhibitors are more effective than a single pathway inhibitor.

In this example the biochemical assay was performed wherein the results are presented as the percent inhibition of activity. Methods employed in this example were adapted from the scientific literature to maximize reliability and reproducibility. See G. W. Carter et al, 5-Lipoxygenase inhibitory activity of zileuton, 256(3) J. Pharmacol Exp. Ther. 929-37 (1991); H. Safayhi et al., Concentration-dependent potentiating and inhibitory effects of Boswellia extracts on 5-Lipoxygenase product formation in stimulated PMNL, 66 Planta Medica. 110-13 (2000). Reference standards were run as an integral part of each assay to ensure the validity of the results obtained.

In this study, the 5-LOX source was human peripheral blood mononuclear cells (PBMNC) and the substrate was arachidonic acid having a general reaction of: arachidonic acid →5-LOX→5-HPETE→LTB₄. Human peripheral blood mononuclear leukocytes (PBML) were isolated through a Ficoll-Paque density gradient. The inhibiting agent was processed *Morinda citrifolia* puree juice concentrate. *Morinda citrifolia* was pre-incubated with PBML (5x10⁶ cell/ml) in HBSS buffer pH 7.4 at 37°C for 15 minutes. The reaction is initiated by addition of 30 µM A 23187 for another 15 minutes incubation period and is then terminated by 1 N HCl. Following neutralization with NaOH and centrifugation, the supernatant LTB₄ is measured using an EIA kit. The results of the study indicated that processed *Morinda citrifolia* puree product, as described and taught herein, functioned as a significant inhibitor.

In regards to the 5-LOX pathways, the *Morinda citrifolia* puree functioned to inhibit the 5-LOX enzyme, which resulted in the inhibition of the oxygenation of arachidonic acid into its intermediate leukotriene constituents, such as LTA₄ hydrolase, LTA₄, and LTC₄ synthetase. In one specific study, introduction of a one percent (1%) *Morinda citrifolia* puree juice concentrate provided a one hundred and three percent (103%) inhibition of 5-LOX enzyme.

Based on these results, it appears that the synergistic inhibition of 5-LOX using one or more *Morinda citrifolia* products provides a promising way to treat and prevent diseases, including inflammatory diseases with little or no stomach irritation by lowering or inhibiting the production of inflammatory mediators, such as LTA₄, LTB₄, LTC₄, etc. As such, it is proposed that the *Morinda citrifolia* products described herein possess significant anti-inflammatory properties.

### Example Two: Morinda citrifolia Alcohol Supernatant Inhibition of 5-LOX

In another example, one liter of processed Morinda citrifolia puree juice was mixed well with one liter of 200 Proof Ethanol for about 20 minutes, centrifuged, and the supernatant was kept but the precipitate was discarded. Again, the first supernatant was mixed well with the same volume of 200 Proof Ethanol for 20 minutes, centrifuged, and the supernatant (second supernatant) was kept but the precipitate was discarded. The alcohol was removed from the second supernatant using the RotoVap with pressure until a brown syrup-like substance was obtained. HPLC was used to verify that samples were alcohol free.

The supernatant sample was utilized in same assay and protocols as outlined in Example One above, wherein the brown substance obtained was substituted for *Morinda citrifolia* puree juice. The alcohol supernatant fraction caused one hundred and two percent (102%) inhibition of activity of 5-LOX (See table below).

| **Assay Name** | **PT#** | **Conc.** | | **Product(pg/well)** | **%Inh.** | | **Avg. %Inh.** |
|---|---|---|---|---|---|---|---|
| | | | | | | | |
| 5-Lipoxygenase | Minimum | | | 98.5 | | | |
| | | | | 112.3 | | | |
| | Maximum | | | 3106.1 | | | |
| | | | | 3520.7 | | | |
| | *Morinda* | | | 47.6 | | 101.8 | 102 |
| | *citrifolia* | | | | | | |
| | (Supernatant) | 1 | % | | | | |
| | *Morinda* | | | 28.1 | | 102.4 | |
| | *citrifolia* | | | | | | |
| | (Supernatant) | 1 | % | | | | |
| | Minimum | | | 112 | | | |
| | | | | 136.7 | | | |
| | Maximum | | | 3910 | | | |
| | | | | 3564.1 | | | |
| | NDGA | 0.03 | uM | 3608.3 | | 3.6 | 6 |
| | NDGA | 0.03 | uM | 3403.4 | | 9.2 | |
| | NDGA | 0.1 | uM | 2433.3 | | 36.1 | 36 |
| | NDGA | 0.1 | uM | 2433.3 | | 36.1 | |
| | NDGA | 0.3 | uM | 1083.1 | | 73.5 | 73 |
| | NDGA | 0.3 | uM | 1106.1 | | 72.8 | |
| | NDGA | 1 | uM | 233.7 | | 97.0 | 97 |
| | NDGA | 1 | uM | 217.9 | | 97.4 | |
| | NDGA | 3 | uM | 134.9 | | 99.7 | 100 |
| | NDGA | 3 | uM | 94.3 | | 100.8 | |
| NDGA= nordihydroguaretic acid | | | | | | | |

Based on these results, it appears that the synergistic inhibition of 5-LOX using one or more *Morinda citrifolia* alcohol supernatant products provides a promising way to treat and prevent inflammatory diseases with little or no stomach irritation by lowering or inhibiting the production of inflammatory mediators, such as LTA₄, LTB₄, LTC₄, etc. As such, it is proposed that the *Morinda citrifolia* products described herein possess significant anti-inflammatory properties.

### Example Three: Morinda citrifolia Puree Juice and Alcohol Supernatant Inhibition of 15-LOX

In another example a biochemical assay was performed wherein the results are presented as the percent inhibition of activity of 15-LOX by *Morinda citrifolia* Puree Juice and Alcohol Supernatant. Methods employed in this example were adapted from the scientific literature to maximize reliability and reproducibility. See B. J. Auerbach et al, A spectrophotometric mictrotiter-based assay for the detection of hydroperoxy derivative of linoleic acid, 201 Anal Biochem. 375-380 (1992).

15-Lipoxygenase initiates the metabolic pathway leading to the formation of lipoxins by converting arachidonic acid to 15-hydroxyperoxy-eicosatetraenoic acid (15-HPETE). Lipoxins may share some of the bioactivity associated with leukotrienes. 15-Lipoxygenase obtained from rabbit reticulocytes was used. Test compound was pre-incubated with 167 U/ml 15-LOX in phosphate buffer pH 7.4 at 4°C for 15 minutes. The reaction was initiated by addition of 256 µM linoleic acid for another 10 minutes incubation period and is then terminated by addition of N-benzoyl leucomethylene blue (LMB) and levels of 15-HETE are determined by measuring absorbance at 660 nm. Results are displayed in the table below. Introducing one percent (1%) *Morinda citrifolia* puree juice concentrate provided an average two hundred and eighty three percent (283%) inhibition of 15-LOX. See table below.

| **Assay Name** | **PT#** | **Conc.** | | **Product(pg/well)** | | **%Inh.** | **Avg. %Inh.** |
|---|---|---|---|---|---|---|---|
| 15-Lipoxygenase | Minimum | | | 0.0116 | | | |
| | | | | 0.0168 | | | |
| | Maximum | | | 0.1347 | | | |
| | | | | 0.1401 | | | |
| | *Morinda citrifolia* (Supernatant) | 1 | % | -0.193 | | 268.2 | 283 |
| | *Morinda citrifolia* (Supernatant) | 1 | % | -0.23 | | 298.2 | |
| | NDGA | 0.1 | uM | 0.119 | | 14.9 | 13 |
| | NDGA | 0.1 | uM | 0.1234 | | 11.4 | |
| | NDGA | 0.3 | uM | 0.1187 | | 15.2 | 20 |
| | NDGA | 0.3 | uM | 0.1073 | | 24.4 | |
| | NDGA | 1 | uM | 0.0816 | | 45.3 | 45 |
| | NDGA | 1 | uM | 0.0825 | | 44.6 | |
| | NDGA | 3 | uM | 0.0272 | | 89.4 | 92 |
| | NDGA | 3 | uM | 0.0217 | | 93.9 | |
| | NDGA | 10 | uM | 0.0117 | | 102.0 | 102 |
| | NDGA | 10 | uM | 0.0109 | | 102.7 | |
| NDGA= nordihydroguaretic acid | | | | | | | |

Based on these results, it appears that the synergistic inhibition of 15-LOX using one or more *Morinda citrifolia* products provides a promising way to treat and prevent disease, including inflammatory diseases with little or no stomach irritation by lowering or inhibiting the production of inflammatory mediators, such as LTA₄, LTB₄, LTC₄, etc. As such, it is proposed that the *Morinda citrifolia* products described herein possess significant anti-inflammatory properties.

The present invention may be embodied in other specific forms without departing from its spirit of essential characteristics. The described embodiments are to be considered in all respects only as illustrative and not restrictive. The scope of the invention is, therefore, indicated by the appended claims, rather than by the foregoing description. All changes which come within the meaning and range of equivalency of the claims are to be embraced within their scope.

## Claims

1. A Lipoxygenase inhibitor for treating inflammatory diseases and symptoms in mammals comprising:
a naturaceutical composition comprising at least one processed *Morinda citrifolia* product selected from the group consisting of processed *Morinda citrifolia* fruit juice, processed *Morinda citrifolia* puree juice, processed *Morinda citrifolia* dietary fiber, processed *Morinda citrifolia* oil, processed *Morinda citrifolia* oil extract and an alcohol soluble fraction isolated and obtained from said processed *Morinda citrifolia* fruit; and
wherein said processed *Morinda citrifolia* product of said naturaceutical formulation further comprises an active ingredient Quercetin present in an amount between about 0.1 and 10 percent by weight.

2. The Lipoxygenase inhibitor of claim 1, wherein said processed *Morinda citrifolia* product is present in an amount between about 0.01 and 99 percent by weight.

3. The Lipoxygenase inhibitor of claim 1, wherein said processed *Morinda citrifolia* product further comprises Rutin as an additional active ingredient.

4. A method for isolating an active ingredient in a processed *Morinda citrifolia* product and using said active ingredient to inhibit Liopxygenase, said method comprising the step of:
obtaining an amount of processed *Morinda citrifolia* puree;
obtaining an amount of ethanol alcohol
mixing said *Morinda citrifolia* puree with said ethanol alcohol in a centrifuge for an identified duration of time;
collecting said alcohol soluble fraction;
removing any residual alcohol from said alcohol soluble fraction to obtain an alcohol soluble fraction active ingredient;
mixing said active ingredient into a naturaceutical formulation;
introducing into said mammal said naturaceutical formulation, wherein said active ingredient functions to inhibit Lipoxygenase and the metabolizing and synthesis of arachidonic acid for the purpose of treating and preventing inflammatory diseases and the symptoms associated therewith.
